# EUROPEAN PATENT APPLICATION

(11) **EP 2 796 135 A1**
(43) Date of publication of application: **29.10.2014**
(21) Application number: 13164730.7
(22) Date of filing: 22.04.2013
(51) Int. Cl.: A61K 31/19, A61P 3/00, A61P 9/00, A61P 9/10

(54) **Use of trifluoroacetic acid and salts thereof to treat hypercholesterolemia**

(71) Applicant: POLICHEM S.A., 1526 Luxembourg (LU)
(72) Inventor: Bulgheroni, Anna, 21100 VARESE (IT); Legora, Michela, 22070 Appiano Gentile (CO) (IT); Ceriani, Daniela, 21050 BESANO (VA) (IT); Mailland, Federico, 6900 LUGANO (CH)
(74) Representative: Minoja, Fabrizio

(57) **Abstract**

The present invention is directed to Trifluoroacetic acid and its physiologically acceptable salts for use in the treatment of hypercholesterolemia.

## Description

The present invention relates to trifluoroacetic acid and its physiologically acceptable salts, for use in the treatment of hypercholesterolemia.

### BACKGROUND OF THE INVENTION

Cholesterol is the main sterol synthesized by animals. It is an essential structural component of the mammalian cell membrane which serves to regulate its permeability and fluidity. Cholesterol also functions in intracellular transport, cell signalling and nerve conduction. It is also an important precursor molecule for the synthesis of Vitamin D and steroid hormones (e.g. cortisol, aldosterone, progesterone, estrogens, testosterone and their derivatives).

Even though all kind of animal cells are able to produce cholesterol, 20 to 25% of the total daily cholesterol production occurs in the liver, the rest being produced mainly by intestines, adrenal glands and reproductive organs.

The biosynthesis of cholesterol is mainly regulated inside the cell by the protein SREBP (sterol regulatory element-binding protein 1 and 2) in the endoplasmic reticulum.

Since cholesterol is almost insoluble in water, it is transported in the circulatory system within different kind of lipoproteins: chylomicrons, very low-density lipoprotein (VLDL), intermediate-density lipoprotein (IDL), low-density lipoprotein (LDL) and high-density lipoprotein (HDL). Among those, LDL is the major carrier of cholesterol in the blood (1500 molecules of cholesterol per LDL molecule). LDL mediates the endocytosis of cholesterol via specific cell membrane LDL receptors. When intracellular levels of cholesterol are high the LDL receptor synthesis is blocked and cholesterol transport inside the cell is impaired.

Cholesterol charged LDL molecules which remain in the blood stream are phagocyted by macrophages which often become trapped in the blood vessels, contributing to the development of atherosclerosis. This process may have as a consequence coronary heart disease, including myocardial infarction, other vascular diseases like stroke and/or peripheral vascular disease.

Hypercholesterolemia can be due to a combination of environmental and genetic factors or can be secondary to other causes like, among the others, obesity, type 2 diabetes mellitus, alcohol abuse and certain medications (beta blockers, retinoic acid, glucocorticoids, cyclosporine and certain diuretics).

The treatment of hypercholesterolemia usually involves a combination of lifestyle modification (no-smoking, limited alcohol consumption, increased physical activity, healthy weight and diet low in saturated fats and low cholesterol food) and medication (statins or in case statins are not tolerated, fibrates, nicotinic acid and cholestyramine).

Although generally safe, statins may cause adverse reactions in specific groups of patients: age > 70 (women at higher risk than man), frailty and small body frame, multisystem disease, concomitant medications (fibrates, cyclosporine, azoles, macrolides, protease inhibitors, nefazodone, verapamil and amiodarone), large quantities of grapefruit and alcohol abuse (ESC/EAS Task force. Guidelines for the management of dyslipidaemias. European Heart Journal, 2011, 32:1769-1818).

The two clinically most important adverse effects associated with statin treatment are hepatotoxicity, reflected by increased levels of alanine aminotransferase (ALT) and aspartate aminotranferase (AST), and miopathy, which includes a range of muscle symptoms and elevated serum markers of muscle injury such as creatine kinase (CK), AST and aldolase (Vasudevan A.R. et al. Cleveland Clinic Journal of Medicine, 2005, 72:990-1000).

Considerable controversies exist on the clinical efficacy of second line therapeutic options to statins (Goldfine A.B. et al. N Engl J Med, 2011, 365:481-484).

As a consequence, there is still an important medical need of safe and effective treatment of hypercholesterolemia.

Trifluoroacetic acid is a small molecule already known as the terminal metabolite of the systemic anaesthetic agent halothane. TFA is not known in therapy, though its sodium salt (NaTFA) is known to be devoid of significant toxicity and its calcium salt is known to inhibit tumor growth in animal experimental models and exert *in vitro* cytotoxic activity on solid tumor cells (WO 03/006031). Calcium trifluoroacetate has also in vitro cytotoxic activity against multiple myeloma, chronic and acute myeloid leukemia human cell lines as well as human marrow stem cells (WO 2006/032458). Calcium trifluoroacetate was also found to display a significant antiangiogenetic activity (Bussolati B. et al. Microvascular Research 2009, 78:272-277) suggesting a potential use for the treatment of atherosclerotic plaque, rheumatoid arthritis, psoriasis, diabetic retinopathy, rosacea and cheloids (WO 2006/000339).

It has now been surprisingly found that trifluoroacetic acid is able to decrease cholesterol blood levels either when orally or intravenously administered.

### DESCRIPTION OF THE INVENTION

The present invention refers to trifluoroacetic acid, or a physiologically acceptable salt thereof, for use in the treatment of hypercholesterolemia.

For the purposes of the present invention, trifluoroacetic acid, or a physiologically acceptable salt thereof, may be suitably formulated for parenteral or oral administration.

Trifluoroacetic acid, or a physiologically acceptable salt thereof, may be formulated in conventional parenteral dosage forms, or in controlled and sustained release drug delivery systems. Conventional parenteral dosage forms includes solutions, either in aqueous, or in oil-based, or in a physiologically acceptable solvent. Controlled and sustained release drug delivery systems include liposomes, microspheres, nanospheres, emulsions, suspensions, gels and implants.

The above formulations for parenteral administration may have a w/w concentration in trifluoroacetic acid, or a physiologically acceptable salt thereof, from 0.5% to 30%, more preferably from 1% to 20%, most preferably from 2% to 10%.

Trifluoroacetic acid, or a physiologically acceptable salt thereof, may be formulated for oral administration as immediate release dosage forms, or as controlled and sustained release drug delivery systems in the form of tablets, film-coated tablets, capsules, soft gel capsules, sachets, solutions, emulsions or suspensions.

The above formulations for oral administration may have a w/w concentration in trifluoroacetic acid, or a physiologically acceptable salt thereof, from 0.5% to 90%, more preferably from 1% to 80%, most preferably from 2% to 70%.

According to an embodiment of the invention, either when administered parenterally or orally, the amount of trifluoroacetic acid or of a physiologically acceptable salt thereof, may vary from 60 to 3000 mg per single dose, more preferably from 120 to 1500 mg per single dose.

Such parenteral or oral formulations are particularly suitable to treat high cholesterol levels in all their manifestations, including high total cholesterol and high LDL cholesterol levels.

All pharmaceutical compositions above may be prepared according to conventional techniques, may contain pharmaceutically acceptable excipients, adjuvants and/or carriers, and may also contain, in combination, one or more active principles with complementary or, in any case, useful activity.

The active agents which may be used in combination with trifluoroacetic acid or a physiologically acceptable salt thereof of the present invention include, but are not limited to, statins, fibrates, nicotinic acid and cholestyramine; said active ingredients may be administered together with trifluoroacetic acid or a physiologically acceptable salt thereof (i.e. they may be for instance contained in the same composition as trifluoroacetic acid or a physiologically acceptable salt thereof) or they may be administered separately from or in temporal proximity with trifluoroacetic acid or a physiologically acceptable salt thereof, either by oral or parenteral route.

Examples of statins include atorvastatin, fluvastatin, lovastatin, mevastatin, pitavastatin, pravastatin, simvastatin; examples of fibrates include clofibrate, bezafibrate, fenofibrate, gemfibrozil, ciprofibrate.

Examples of the compositions according to the invention include: tablets, film-coated tablets, capsules, solution, granules, effervescent granules, syrup suitable for oral administration, ampoules and vials for parenteral administration.

The pharmaceutical compositions and the uses of the present invention will now be more fully described by the following examples.

### EXAMPLE 1

Solutions for oral administration having the following composition were prepared:

| | |
|---|---|
| 1. Calcium trifluoroacetate | 500-1000-1500-2000 mg |
| 2. water for injection | q.s. to 10 mL |

### Preparation

Calcium trifluoroacetate was dissolved in water and the mix was stirred till to obtain a clear and colourless solution, homogenous in appearance.

### EXAMPLE 2

A liquid formulation for parenteral administration having the following w/V % composition was prepared:

| | |
|---|---|
| 1. Calcium Trifluoroacetate | 10.00% |
| 2. Sodium Chloride | 0.90% |
| 3. water for injection | 89.10% |

### Preparation

Calcium trifluoroacetate and sodium chloride were dissolved in water and the mix was stirred till to obtain a clear solution. The obtained formulation was a clear and colourless solution, homogenous in appearance.

### EXAMPLE 3

A tablet for oral administration having the following w/w % composition was prepared:

| | |
|---|---|
| 1. Calcium Trifluoroacetate | 75.000% |
| 2. Fumed silica, hydrophilic | 1.125% |
| 3. Poly(ethylene glycol) 6000, powder | 0.750% |
| 4. Cellulose, microcrystalline | 13.500% |
| 5. Polyvinylpyrrolidone, crosslinked | 3.375% |
| 6. Vinylpyrrolidone-vinyl acetate copolymer | 6.250% |

### Preparation

Calcium trifluoroacetate was mixed with 2) to 6) listed ingredients. The mixture was passed through a 0.8 mm sieve prior to the compression. Compression was performed by means of a tableting machine using medium to low compression force. The obtained tablet was a round, smooth surface, white tablet.

### EXAMPLE 4

An enteric coated tablet for oral administration was prepared. Core composition:

| | |
|---|---|
| 1. Calcium Trifluoroacetate | 60.000% |
| 2. Cellulose, microcrystalline | 32.500% |
| 3. Polyvinylpyrrolidone, crosslinked | 3.250% |
| 4. Polyvinylpyrrolidone | 3.250% |
| 5. Magnesium stearate | 1.000% |

| Coating suspension composition: | |
|---|---|
| 6. Methacrylic acid ethyl acrylate copolymer | 7.500% |
| 7. Triethyl citrate | 0.750% |
| 8. Ethyl alcohol | 91.750% |

### Preparation

Calcium trifluoroacetate was mixed with 2 to 5 listed core ingredients. The mixture was passed through a 0.8 mm sieve prior to the compression. Compression was performed by means of a tabletting machine using medium to low compression force. The obtained core was a convex, smooth surface, white tablet.

In a separate pan, methacrylic acid ethyl acrylate copolymer and triethyl citrate were mixed with ethyl alcohol until compete dissolution to obtain a coating solution. The coating solution was applied to the cores by means of spray device in a coating pan. The final weight gain was approximately 7.5%. The obtained coated cores were round in shape and smooth surfaced.

### EXAMPLE 5

A capsule, filled with granulates, for oral administration having the following w/w % composition was prepared.

### Core composition:

| | |
|---|---|
| 1. Calcium Trifluoroacetate | 60.000% |
| 2. Cellulose, microcrystalline | 32.500% |
| 3. Polyvinylpyrrolidone, crosslinked | 3.250% |
| 4. Polyvinylpyrrolidone | 3.250% |
| 5. Magnesium stearate | 1.000% |

### Preparation

Calcium trifluoroacetate was mixed with 2 to 5 listed ingredients. The mixture was passed through a 0.8 mm sieve to obtain a granulate. Capsules were filled by means of a filling machine.

### EXAMPLE 6

A dry syrup formulation for oral administration having the following w/w % composition was prepared:

| | |
|---|---|
| 1. Calcium Trifluoroacetate | 20.00% |
| 2. Sodium citrate | 10.00% |
| 3. Citric acid | 3.20% |
| 4. Sodium gluconate | 10.00% |
| 5. Sorbitol crystalline | 44.00% |
| 6. Polyvinylpyrrolidone, crosslinked | 9.20% |
| 7. Orange flavor | 2.30% |
| 8. Lemon flavor | 0.70% |
| 9. Saccharine sodium | 0.60% |

### Preparation

Calcium trifluoroacetate was mixed together with components 2 to 9 and an homogeneous powder was obtained. To 50 g of the dry powder, water was added to 100 mL volume. An homogeneous solution containing Calcium trifluoroacetate at a concentration of 10% w/V was obtained.

### EXAMPLE 7

An effervescent granulate for oral administration was prepared.

*Preparation.*

| Mix I composition | |
|---|---|
| 1. Calcium Trifluoroacetate | 21.500% |
| 2. Sodium bicarbonate | 32.150% |
| 3. Tartaric acid | 43.000% |
| 4. Polyvinylpyrrolidone, water soluble | 3.350% |

| Mix II composition | |
|---|---|
| 5. Polyvinylpyrrolidone, water soluble | 5.000% |
| 6. Isopropyl alcohol | 95.000% |

### Preparation

### Mix I.

Calcium trifluoroacetate was mixed together with components 2 to) and an homogeneous powder was obtained.

### Mix II.

water soluble Polyvinylpyrrolidone was dissolved in Isopropyl alcohol.

Mix I was granulated using Mix II in a fluid bed granulator or, alternatively, a high shear mixer. Prior to partition into sachets by means of a suitable filling machine, the obtained granulate was mixed with a suitable quantity of Crystalline Sucrose in order to obtain a weight gain from 20 to 25%.

### EXAMPLE 8

A granulate for oral administration having the following w/w % composition was prepared:

| | |
|---|---|
| 1. Trifluoroacetic acid, Calcium salt | 26.67% |
| 2. Mannitol | 3.33% |
| 3. Binder and wetting agent | 0.90% |
| 4. Sweetener | 0.60% |
| 5. Flavour | 16.67% |
| 6. Sodium carbonate anhydrous | 5.67% |
| 7. Silicon dioxide | 0.33% |
| 8. Colouring agents | 0.04% |
| 9. Saccharose | q.s. to 100% |

### Preparation

In a vessel dissolve the component 3 in a suitable quantity of water. Mix until clear solution. In another vessel mix the components 1 and 2. Spray the obtained solution onto mixed components until a homogeneous granulate is obtained. After drying, components from 4 to 9 are added to the obtained granulate. All components are mixed until an homogeneous mixture is obtained.

### EXAMPLE 9

An injectable solution having the following w/V composition was prepared:

| | |
|---|---|
| 1. Calcium trifluoroacetate | 500 mg |
| 2. Water for injections | q.s. to 3.3 mL |

Preparation: Calcium trifluoroacetate was dissolved in water for injections and mixed until a homogeneous, clear and colorless solution was obtained.

### EXAMPLE 10

An injectable solution having the following w/V composition was prepared:

| | |
|---|---|
| 1. Calcium trifluoroacetate | 500 mg |
| 2. Water for injections | q.s. to 3.3 mL |
| 3. Sodium Hydroxide | q.s. to pH 7.4 |

Preparation: Calcium trifluoroacetate was dissolved in water for injections and mixed until a homogeneous solution was obtained, then NaOH is added until a pH of 7.4 was reached. The obtained formulation was a clear and colourless solution, homogenous in appearance.

### EXAMPLE 11

A non-aqueous injectable formulation was prepared having the following composition:

| | |
|---|---|
| 1. Calcium trifluoroacetate | 10% |
| 2. Benzyl alcohol | 3% |
| 3. Polysorbate 80 | 8% |
| 4. PEG 300 | 57% |
| 5. Ethyl alcohol 35% (v/V) | 22% |

Preparation: Calcium trifluoroacetate was dissolved in the mixture benzyl alcohol and polysorbate 80. PEG 300 was added, followed by ethanol. The obtained formulation was a clear and colourless solution, homogenous in appearance.

### EXAMPLE 12

A colloidal carrier system formulation was prepared having the following composition:

| | |
|---|---|
| 1. Calcium trifluoroacetate | 20% |
| 2. PEG 400 | 40% |
| 3. Soybean oil | 5% |
| 4. Phosphatidylcholine | 2.5% |
| 5. Tween 80 | 20% |
| 6. Water for Injection | 12.5% |

Preparation: Calcium trifluoroacetate was dissolved in PEG 400 by vortexing. Thereafter oil was mixed, followed by surfactant and phospholipid. The resulting solution was mixed uniformly by vortexing. Then the fixed amount of water was added and gently mixed to obtain emulsion.

### EXAMPLE 13

A preliminary oral study of the Calcium salt of trifluoroacetic acid was performed in minipigs. One male and one female Göttingen minipig were daily treated by oral route with ascending dose levels of Calcium salt of trifluoroacetic acid, as indicated below.

| | **Dose level (mg/kg/day)** | **Dose Volume (ml/kg)** | **Solution concentration (mg/ml)** | **Days of treatment** |
|---|---|---|---|---|
| Step 1 | 50 | 1 | 50 | 1-3 |
| Step 2 | 100 | 1 | 100 | 4-6 |
| Step 3 | 150 | 1 | 150 | 7-9 |
| Step 4 | 200 | 2 | 100 | 10-13 |
| Step 5 | 300 | 2 | 150 | 14-17 |
| Step 6 | 600 | 4 | 150 | 18-20 |
| Wash out | / | / | / | 21-24 |
| Step 7 | 1000 | 5 | 200 | 25-28 |

The oral solutions were prepared as for Example 1.

No mortality occurred during the study. No changes of toxicological relevance were recorded in body weight, food consumption and water consumption. No changes of note were detected in hematology. The maximum tolerated dose was set at ≥ 1000 mg/kg.

A dose-related significant decrease of total cholesterol was observed in the male starting from 150 mg/kg/day onwards: 18% for the dose of 150 mg/kg/day, 33% for 200 mg/kg/day, 44% for 300 mg/kg/day, 49% for 600 mg/kg/day and 56% for 1000 mg/kg/day.

### EXAMPLE 14

A preliminary intravenous study of the Calcium salt of trifluoroacetic acid was performed in Beagle dogs after daily administration.

Dogs (2 animals/sex/group) were treated by intravenous slow injection (2.5 mL/min) with 100 and 200 mg/kg for 14 consecutive days.

The intravenous solutions were prepared as for Example 9.

No deaths occurred during the study.

No significant differences were recorded in body weight, food consumption, physical examination and chemical chemistry, coagulation, organ weight. The only findings recorded in this study were limited to the site of injection.

A decrease in total cholesterol was observed in both sexes at both tested doses (100 and 200 mg/kg/day) at the end of the 14 day of treatment. A reduction by 29% and 16% in females and by 18% and 21% in males was observed at the doses of 100 mg/kg/day and 200 mg/kg/day, respectively.

### EXAMPLE 15

An intravenous study of the Calcium salt of trifluoroacetic acid was performed in Beagle dogs after daily administration for 4 weeks.

Dogs (4 animals/sex/group) were treated by intravenous slow injection (2.5 mL/min) with 50, 100 and 200 mg/kg for 4 consecutive weeks.

The intravenous solutions were prepared as for Example 9.

No deaths occurred during the study.

No changes of toxicological relevance were recorded in body weight, food consumption and water consumption as well as in hematological, chemical chemistry, fecal and urinalysis parameters of both sexes from treated groups. Organ weights appeared to have been unaffected.

Minimal to moderate findings recorded at the site of injection and electrographic alterations observed were dose dependent and reversible.

A decrease in total cholesterol was observed in both sexes at all tested doses (50, 100 and 200 mg/kg/day) at the end of the 4 weeks of treatment. A reduction by 20%, 29% and 24% in females and by 9% and 21% and 15% in males was observed at the doses of 50 mg/kg/day, 100 mg/kg/day and 200 mg/kg/day, respectively.

## Claims

1. Trifluoroacetic acid or a physiologically acceptable salt thereof, for use in the treatment of hypercholesterolemia.

2. Trifluoroacetic acid or a physiologically acceptable salt thereof for use according to claim 1, **characterized in that** said hypercholesterolemia is high total cholesterol or high LDL cholesterol levels.

3. Trifluoroacetic acid or a physiologically acceptable salt thereof for use according to claim 1, **characterized in that** it is administered orally.

4. Trifluoroacetic acid or a physiologically acceptable salt thereof for use according to claim 3, **characterized in that** it is administered by means of a solid or liquid formulation.

5. Trifluoroacetic acid or a physiologically acceptable salt thereof for use according to claim 4, **characterized in that** said solid formulation is a tablet, a film-coated tablet, a capsule, a granule, a soft-gel capsule or a sachet.

6. Trifluoroacetic acid or a physiologically acceptable salt thereof for use according to claim 4, **characterized in that** said liquid formulation is a solution, an emulsion or a suspension.

7. Trifluoroacetic acid or a physiologically acceptable salt thereof for use according to claim 1, **characterized in that** it is administered parenterally.

8. Trifluoroacetic acid or a physiologically acceptable salt thereof for use according to claim 7, **characterized in that** it is administered by means of a conventional parenteral dosage form or a controlled and sustained release drug delivery system.

9. Trifluoroacetic acid or a physiologically acceptable salt thereof for use according to claim 8, **characterized in that** said conventional parenteral dosage form is either in aqueous, or in oil-based, or in a physiologically acceptable solvent.

10. Trifluoroacetic acid or a physiologically acceptable salt thereof for use according to claim 8, **characterized in that** said controlled or sustained release drug delivery systems are selected from liposomes, microspheres, nanospheres, emulsions, suspensions, gels or implants.

11. Trifluoroacetic acid or a physiologically acceptable salt thereof for use according to claims 3 and 7, **characterized in that** said solid or liquid formulation has a w/w concentration in trifluoroacetic acid or physiologically acceptable salt thereof from 0.5% to 90%, more preferably from 1% to 80%, most preferably from 2% to 70%.

12. Trifluoroacetic acid or a physiologically acceptable salt thereof for use according to claim 4, **characterized in that** said formulation has a content in trifluoroacetic acid or a salt thereof, from 60 to 3000 mg per single dose, more preferably from 120 to 1500 mg.

13. Calcium trifluoroacetate for use according to any one of the preceding claims.

14. Trifluoroacetic acid or a physiologically acceptable salt thereof for use according to any of the preceding claims, **characterized in that** it is administered in combination or in temporal proximity with at least one additional active principle, preferably selected from statins, fibrates, nicotinic acid, cholestyramine.

15. Trifluoroacetic acid or a physiologically acceptable salt thereof for use according to claim 14, **characterized in that** said statin is selected from atorvastatin, fluvastatin, lovastatin, mevastatin, pitavastatin, pravastatin and simvastatin and said fibrate is selected from clofibrate, bezafibrate, fenofibrate, gemfibrozil and ciprofibrate.
